# EUROPEAN PATENT SPECIFICATION

(11) **EP 2 844 644 B1**
(45) Date of publication and mention of the grant of the patent: **14.11.2018**
(21) Application number: 13720372.5
(22) Date of filing: 02.05.2013
(51) Int. Cl.: C07D 263/34, C07C 59/215, C07C 67/28, C07C 67/31, C07C 69/06, C07C 69/716

(54) **PROCESS FOR PREPARATION OF 4-METHYLOXAZOLE-5-CARBOXYLIC ESTER**
VERFAHREN ZUR HERSTELLUNG VON 4-METHYLOXAZOL-5-CARBONSÄUREESTER
PROCÉDÉ DE PRÉPARATION D'ESTERS CARBOXYLIQUES DE 4-METHYLOXAZOLE

(30) Priority: 03.05.2012 EP 12166591
(43) Date of publication of application: 11.03.2015
(60) Divisional application: 17182310.7
(73) Proprietor: DSM IP Assets B.V., 6411 TE Heerlen (NL)
(72) Inventor: EISELE, Frank, CH-4002 Basel (CH)
(74) Representative: Kurt, Manfred
(86) International application number: PCT/EP2013/059086
(87) International publication number: WO 2013/185976

(56) References cited:
- EP-A1- 0 220 573
- EP-A1- 0 220 573
- WO-A1-2012/109100
- WO-A1-2012/109100
- WO-A1-2012/109100
- GB-A- 1 579 201
- GB-A- 1 579 201
- US-A1- 2007 105 905
- US-A1- 2007 105 905
- US-A1- 2007 105 905
- Z. I. ITOV ET AL: "SYNTHESIS OF 4-METHYL-S-ETHOXYCARBONYLOXAZOLE", PHARMACEUTICAL CHEMISTRY JOURNAL, vol. 33, no. 4, 1 January 1989 (1989-01-01), pages 330-332, XP055066289,
- ALTUNA-URQUIJO M ET AL: "A convenient synthesis of pyridine and 2,2'-bipyridine derivatives", TETRAHEDRON, ELSEVIER SCIENCE PUBLISHERS, AMSTERDAM, NL, vol. 65, no. 5, 31 January 2009 (2009-01-31), pages 975-984, XP025816077, ISSN: 0040-4020, DOI: 10.1016/J.TET.2008.11.090 [retrieved on 2008-12-03]
- MITSURU KITAMURA ET AL: "Synthesis of 1,2-Naphthalenediol Diacetates by Rhodium(II)-Catalyzed Reaction of 1,2-Diazonaphthoquinones with Acetic Anhydride", EUROPEAN JOURNAL OF ORGANIC CHEMISTRY, vol. 2012, no. 5, 9 February 2012 (2012-02-09), pages 905-907, XP055066385, ISSN: 1434-193X, DOI: 10.1002/ejoc.201101698
- GÜNTHER SCHEID ET AL: "A New Route to Protected Acyloins and Their Enzymatic Resolution with Lipases", EUROPEAN JOURNAL OF ORGANIC CHEMISTRY, vol. 2004, no. 5, 1 March 2004 (2004-03-01), pages 1063-1074, XP055066388, ISSN: 1434-193X, DOI: 10.1002/ejoc.200300338
- TAKUYA HASHIMOTO ET AL: "Phase-Transfer-Catalyzed Asymmetric Alkylation of [alpha]-Benzoyloxy-[beta]-keto Esters: Stereoselective Construction of Congested 2,3-Dihydroxycarboxylic Acid Esters", CHEMISTRY - AN ASIAN JOURNAL, vol. 5, no. 3, 1 March 2010 (2010-03-01), pages 562-570, XP055066391, ISSN: 1861-4728, DOI: 10.1002/asia.200900344
- KHARCHUK V G ET AL: "Reaction of 1,2,4-trimethylbenzene with peracetic acid", JOURNAL OF ORGANIC CHEMISTRY OF THE USSR = ZHURNAL ORGANICHESKOI KHIMII, M A I K NAUKA - INTERPERIODICA, RU, vol. 22, 1 January 1986 (1986-01-01), pages 2071-2078, XP009170338, ISSN: 0022-3271
- "Compound Summary for CID 21391690", PubChem Compound, 5 December 2007 (2007-12-05), pages 1-11, XP055262962, Retrieved from the Internet: URL:http://pubchem.ncbi.nlm.nih.gov/compou nd/21391690#section=Top [retrieved on 2016-04-05]
- H BREDERECK ET AL: "Neuere Methoden der präparativen organischen Chemie II: 16. Synthesen mit Säure-arniden, insbesondere mit Formamid", ANGEWANDTE CHEMIE, vol. 71, no. 24, 1 January 1959 (1959-01-01), pages 753-774, XP055262338,
- BREDERECK H ET AL: "Oxazol-Synthesen aus alpha-Halogen-ketonen", CHEMISCHE BERICHTE, VCH, DE, vol. 87, no. 5, 1 January 1954 (1954-01-01), pages 700-707, XP002148209, ISSN: 0009-2940
- TAKASHI SAKAI ET AL: BULLETIN OF THE CHEMICAL SOCIETY OF JAPAN, vol. 59, no. 10, 1 January 1986 (1986-01-01), pages 3185-3188, XP055262624,
- Z. I. ITOV ET AL: "SYNTHESIS OF 4-METHYL-S-ETHOXYCARBONYLOXAZOLE", PHARMACEUTICAL CHEMISTRY JOURNAL, vol. 33, no. 4, 1 January 1989 (1989-01-01), pages 330-332, XP055066289,
- ALTUNA-URQUIJO M ET AL: "A convenient synthesis of pyridine and 2,2'-bipyridine derivatives", TETRAHEDRON, ELSEVIER SCIENCE PUBLISHERS, AMSTERDAM, NL, vol. 65, no. 5, 31 January 2009 (2009-01-31), pages 975-984, XP025816077, ISSN: 0040-4020, DOI: 10.1016/J.TET.2008.11.090 [retrieved on 2008-12-03]
- MITSURU KITAMURA ET AL: "Synthesis of 1,2-Naphthalenediol Diacetates by Rhodium(II)-Catalyzed Reaction of 1,2-Diazonaphthoquinones with Acetic Anhydride", EUROPEAN JOURNAL OF ORGANIC CHEMISTRY, vol. 2012, no. 5, 9 February 2012 (2012-02-09), pages 905-907, XP055066385, ISSN: 1434-193X, DOI: 10.1002/ejoc.201101698
- GÜNTHER SCHEID ET AL: "A New Route to Protected Acyloins and Their Enzymatic Resolution with Lipases", EUROPEAN JOURNAL OF ORGANIC CHEMISTRY, vol. 2004, no. 5, 1 March 2004 (2004-03-01), pages 1063-1074, XP055066388, ISSN: 1434-193X, DOI: 10.1002/ejoc.200300338
- TAKUYA HASHIMOTO ET AL: "Phase-Transfer-Catalyzed Asymmetric Alkylation of [alpha]-Benzoyloxy-[beta]-keto Esters: Stereoselective Construction of Congested 2,3-Dihydroxycarboxylic Acid Esters", CHEMISTRY - AN ASIAN JOURNAL, vol. 5, no. 3, 1 March 2010 (2010-03-01), pages 562-570, XP055066391, ISSN: 1861-4728, DOI: 10.1002/asia.200900344
- KHARCHUK V G ET AL: "Reaction of 1,2,4-trimethylbenzene with peracetic acid", JOURNAL OF ORGANIC CHEMISTRY OF THE USSR, M A I K NAUKA - INTERPERIODICA, RU, vol. 22, 1 January 1986 (1986-01-01), pages 2071-2078, XP009170338, ISSN: 0022-3271
- Z. I. ITOV ET AL: "SYNTHESIS OF 4-METHYL-S-ETHOXYCARBONYLOXAZOLE", PHARMACEUTICAL CHEMISTRY JOURNAL, vol. 33, no. 4, 1 January 1989 (1989-01-01), pages 330-332, XP055066289,
- ALTUNA-URQUIJO M ET AL: "A convenient synthesis of pyridine and 2,2'-bipyridine derivatives", TETRAHEDRON, ELSEVIER SCIENCE PUBLISHERS, AMSTERDAM, NL, vol. 65, no. 5, 31 January 2009 (2009-01-31), pages 975-984, XP025816077, ISSN: 0040-4020, DOI: 10.1016/J.TET.2008.11.090 [retrieved on 2008-12-03]
- MITSURU KITAMURA ET AL: "Synthesis of 1,2-Naphthalenediol Diacetates by Rhodium(II)-Catalyzed Reaction of 1,2-Diazonaphthoquinones with Acetic Anhydride", EUROPEAN JOURNAL OF ORGANIC CHEMISTRY, vol. 2012, no. 5, 9 February 2012 (2012-02-09), pages 905-907, XP055066385, ISSN: 1434-193X, DOI: 10.1002/ejoc.201101698
- GÜNTHER SCHEID ET AL: "A New Route to Protected Acyloins and Their Enzymatic Resolution with Lipases", EUROPEAN JOURNAL OF ORGANIC CHEMISTRY, vol. 2004, no. 5, 1 March 2004 (2004-03-01), pages 1063-1074, XP055066388, ISSN: 1434-193X, DOI: 10.1002/ejoc.200300338
- TAKUYA HASHIMOTO ET AL: "Phase-Transfer-Catalyzed Asymmetric Alkylation of [alpha]-Benzoyloxy-[beta]-keto Esters: Stereoselective Construction of Congested 2,3-Dihydroxycarboxylic Acid Esters", CHEMISTRY - AN ASIAN JOURNAL, vol. 5, no. 3, 1 March 2010 (2010-03-01), pages 562-570, XP055066391, ISSN: 1861-4728, DOI: 10.1002/asia.200900344
- KHARCHUK V G ET AL: "Reaction of 1,2,4-trimethylbenzene with peracetic acid", JOURNAL OF ORGANIC CHEMISTRY OF THE USSR = ZHURNAL ORGANICHESKOI KHIMII, M A I K NAUKA - INTERPERIODICA, RU, vol. 22, 1 January 1986 (1986-01-01), pages 2071-2078, XP009170338, ISSN: 0022-3271
- "Compound Summary for CID 21391690", PubChem Compound, 5 December 2007 (2007-12-05), pages 1-11, XP055262962, Retrieved from the Internet: URL:http://pubchem.ncbi.nlm.nih.gov/compou nd/21391690#section=Top [retrieved on 2016-04-05]
- H BREDERECK ET AL: "Neuere Methoden der präparativen organischen Chemie II: 16. Synthesen mit Säure-arniden, insbesondere mit Formamid", ANGEWANDTE CHEMIE, vol. 71, no. 24, 1 January 1959 (1959-01-01), pages 753-774, XP055262338,
- BREDERECK H ET AL: "Oxazol-Synthesen aus alpha-Halogen-ketonen", CHEMISCHE BERICHTE, VCH, DE, vol. 87, no. 5, 1 January 1954 (1954-01-01), pages 700-707, XP002148209, ISSN: 0009-2940
- TAKASHI SAKAI ET AL: BULLETIN OF THE CHEMICAL SOCIETY OF JAPAN, vol. 59, no. 10, 1 January 1986 (1986-01-01), pages 3185-3188, XP055262624,

## Description

### TECHNICAL FIELD

The present invention is related to a new process for the preparation of 4-methyloxazole-5-carboxylic ester, which is a valuable intermediate in the synthesis of pyridoxine (vitamin B₆).

### BACKGROUND OF INVENTION

Several processes for the manufacture of pyridoxine have already been described. A summary of the most important ones is to be found, e.g., in Ullmann's Encyclopedia of Industrial Chemistry, 5th edition, 1996, vol. A 27, p. 533-537. For the industrial synthesis of pyridoxine there is now used nearly exclusively the approach described by Kondratyeva, G.Y., Khim. Nauka Promst. 2, 666 (1957), in which approach the pyridine ring is obtained by a Diels-Alder reaction of oxazoles with maleic acid or its derivatives. A particularly preferred oxazole in the synthetic pathway is 5-cyano-4-methyl-oxazole which is generally prepared from 4-methyloxazole-5-carboxylic ester (see patent publications US 4,772,718 and EP 10697).

Patent publications US 3538110, IN 177708, US 4026901, US2009143346 etc. disclose the process for the preparation of 4-methyloxazole-5-carboxylic ester. US 3538110 discloses a common-used process which comprises reacting ethyl 2-chloro-acetoacetate (EACA) with formamide to give 4-methyloxazole-5-formic acid ethyl ester (OXE) together with additional formic acid and ammonium chloride.

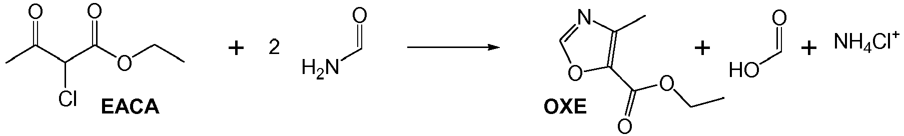

Z. I. Atov *et al.* discloses a process for the preparation of 4-methyl-5-ethoxy-carbonyl-oxazole (I) from ethyl 2-chloroacetoacetate (II). In the process, the maximum yield of (I) were obtained when the oxazole was obtained in two steps. First, ethyl 2-formoxyacetoacetate was obtained by reacting (II) with sodium formate in DMF and then was heated with formamide in the presence of catalystic amounts of sulfuric acid (see Khimiko-farmatsevticheskii Zhurnal, Vol. 23, No. 4, pp. 452-454, April 1989).

However, the process is restricted because it results in poor yields of no more than 65%. In addition, the process has the drawback of a high consumption of expensive formamide. Stoichiometrically two equivalents of formamide are needed, which can add up to 3 eq. or more under real process conditions of the process.

Therefore, there is still a existing need for a new process for the preparation of 4-methyloxazole-5-carboxylic ester.

### SUMMARY OF INENTION

The present invention provides a new process for the preparation of 4-methyloxazole-5-carboxylic ester, which has a high yield of more than 80%, and a lower cost due to low formamide consumption.

The first aspect of the present invention provides an intermediate compound of formula (I), which can be used to prepare 4-methyloxazole-5-carboxylic ester: 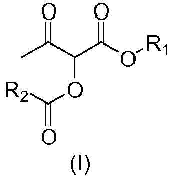 wherein R₁ is H or C₁-C₁₀alkyl; and R₂ is H.

The second aspect of the present invention provides a process for the preparation of 4-methyloxazole-5-carboxylic ester, represented by a compound of formula (II), which comprises reacting the compound of formula (I) with formamide in the presence of an acid catalyst, 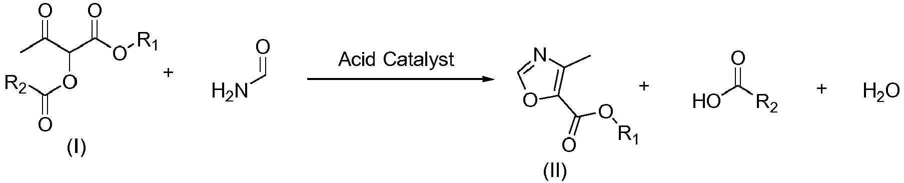 wherein R₁ and R₂ are as defined above.

### DETAILED DESCRIPTION OF INVENTION

In the present invention, the term "C₁-C₁₀ alkyl" as used refers to branched or unbranched, cyclic or non-cyclic, saturated alkyl comprising 1-10 carbon atoms. Preferably, the "C₁-C₁₀ alkyl" is C₁-C₄ alkyl, and includes but is not limited to methyl, ethyl, propyl, isopropyl, cyclopropyl, butyl, isobutyl, tert-butyl, methyl cyclopropyl and cyclobutyl. More preferably, the "C₁-C₁₀ alkyl" is methyl or ethyl.

In the present invention, the term "halo" or "halogen" as used refers to a group of elements including fluorine (F), chlorine (Cl), bromine (Br) and iodine (I), preferably refers to Cl or Br.

In the present invention, the term "alkali metal element" as used refers to the group 1 element excluding hydrogen in the periodic table, including lithium (Li), sodium (Na), potassium (K), rubidium (Rb), Caesium (Cs) and francium (Fr). Preferably, the term "alkali metal element" according to the present invention refers to Na or K.

In the present invention, the term "alkaline-earth metal element" as used refers to the group 2 element in the periodic table, including beryllium (Be), magnesium (Mg), calcium (Ca), strontium (Sr), barium (Ba) and radium (Ra). Preferably, the term "alkali metal element" according to the present invention refers to Mg or Ca.

The first aspect of the present invention provides an compound of formula (I): 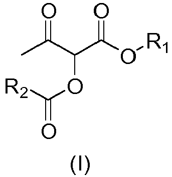 wherein R₁ is H or C₁-C₁₀alkyl; and R₂ is H.

In a preferred embodiment, the compound of formula (I) is 2-formyloxy-3-oxo-butanoic acid methyl ester, or 2-formyloxy-3-oxo-butanoic acid ethyl ester (FOXE). In the most preferred embodiment, the compound of formula (I) is FOXE.

The compound of formula (I) may be used to prepare 4-methyloxazole-5-carboxylic ester, an important intermediate for synthesis of pyridoxine.

Accordingly, the second aspect of the present invention provides a process for the preparation of 4-methyloxazole-5-carboxylic ester, represented by a compound of formula (II), which comprises reacting the compound of formula (I) with formamide in the presence of an acid catalyst, 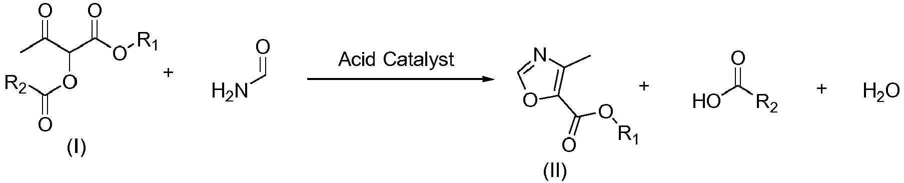 wherein R₁ and R₂ are defined as above.

In an embodiment of the above process, the compound of formula (II) is 4-methyloxazole-5-formic acid methyl ester, or 4-methyloxazole-5-formic acid ethyl ester (OXE).

The acid catalyst useful in the reaction may be any mineral acid or organic acid, preferably high boiling acids such as H₂SO₄ or H₃PO₄, or acidic ion exchange resins. The most preferred acid catalyst useful in the reaction is H₂SO₄.

Formamide may be added into the reaction in any amount. Preferably, the amount of formamide added into the reaction varies from 1.0 mol to 20 mol, more preferably it is kept between 1.0 mol and 10 mol, and most preferably between 1.0 and 2.0 mol, per 1 mol of the compound of formula (I). The amount of the acid catalyst in the reaction may vary from 1 mole% to 10 mole%, and preferably it is in the range of 3 mole% to 8 mole %, based on compound of formula (I).

The reaction may be performed batch-wise, semi-batch-wise or continuously. Preferably, carboxylic acid and water are removed from the reaction mixture during the entire reaction. For example, carboxylic acid and water are removed by distillation, such as rectification.

The reaction may be conveniently carried out under pressure and temperature conditions suitable for the reactions. Preferably, the reaction is carried out at atmospheric or reduced pressure at a temperature between 100°C and 200°C, most preferably an a temperature between 120°C and 140°C.

Optionally, the reaction is carried out under an inert gas atmosphere e.g. under nitrogen or argon or mixtures thereof. Any method known in the art, such as gas chromatography, may be used to monitor the proceeding of the reaction. After the reaction, the resulting compound of formula (II) may be used directly for synthesis of pyridoxine or isolated from the reaction by a simple straight forward method known in the art, such as filtration, distillation, rectification or combination thereof. The remaining formamide can be recycled back to the reaction, while the obtained carboxylic acid may be converted to its metal salts and then used for the preparation of the compound of formula (I), as described in the content below.

Preferably, the reaction is carried out in the absence of a solvent. In this case, the formamide consumption of the reaction can be reduced to 1.3 eq. needed for the reaction, and complex separation and recycling of formamide can be avoided. Further, as mentioned above, the carboxylic acid and water as side products of the reaction can be removed from the compound of formula (II) easily, for example, by distillation, and then recycled. Accordingly, the reaction has an extremely simple subsequent work-up and is cost efficient.

As raw material of the reaction, formamide is commercially available or may be produced by any method known in the art, and the compound of formula (I) may be prepared from 2-halo-acetoacetate by the process as described in the content below.

The whole process for preparing 4-methyloxazole-5-carboxylic ester according to the present invention has a higher selectivity and high yield of more than 80%. In addition, the process has also other advantages including:
- Less formamide usage: in the process, less than 2.0 eq. formamide are consumed, so the process of the invention is cost efficient;
- Less organic waste: in the process, the produced carboxylic acid can be recycled and as a result of the higher yield and selectivity less organic side products are created in the reactions of the process. Also, less formamide is degraded to waste products in the reactions;
- Easy separation of inorganic salt: the metal salts produced in the reactions of the process can be precipitated easily, so less haloid waste such as chloride waste is carried into organic waste streams; and
- Less subsequent work-up: in the process, less side product is produced and less solvent is used, so the subsequent work-up is extremely simplified.

The present invention is illustrated further by the following Examples. These Examples are not intended to limit the invention in any way.

### EXAMPLES

### Example 1: Preparation of 2-formyloxy-3-oxo-butanoic acid ethyl ester (FOXE)

**1710.9** g DMF (**98**%, **22.94** mole, **8.43** eq.), **65.2** g formic acid (**99**%, **1.40** mole, **0.52** eq.) and **203.5** g sodium formate (**98**%, **2.932** mole, **1.08** eq.) were charged into a **3** L-stirred reaction vessel with a **30** mm × **30** cm Kerapak column. The mixture was dried by distillation at 70 mbar and **105**°C jacket temperature. **473.2** g EACA (**94.**7%, **2.72** mole, **1.0** eq.) was added within **10** min. The reaction mixture was reacted at 65 mbar and 85°C for **4** h while distilling off DMF/light boilers. After cooling to **24**°C, the reaction suspension was filtered to give **1911.8** g filtrate. The filter cake is washed with **316.1** g DMF. The washings are combined with the filtrate. **2272.0** g of the combined filtrate and washings are submitted to a batch distillation to remove the solvent. The resulting suspension in the sump is submitted to a filtration to obtain **441.1** g FOXE crude as the filtrate.
Overall yield of FOXE: **88.8**%
Conversion EACA: **100.0**%
Selectivity FOXE: **88.8**%

### Example 2: Preparation of 4-methyloxazole-5-formic acid ethyl ester (OXE)

A **500** mL-stirred reaction vessel with a **30** mm × **30** cm Kerapak column was charged with **150.2** g formamide (**98**%, **3.267** mole, **1.60** eq.) and **13.4** g sulphuric acid (**98**%, **0.135** mole, **0.07** eq.). The mixture was heated at p = **65** mbar to an inner temperature = **120**°C. Then **400.2** g FOXE crude (**88.6**%, **2.036** mole, **1.00** eq.) was added within **5** h.. A HCOOH/water mixture was distilled from the reaction mixture during the FOXE dosage time. After the end of the FOXE dosage HCOOH/water was further distilled of to complete the reaction. This was the case after additional **1.5** h of reaction and distillation after the end of the FOXE addition. During the complete reaction an overall amount of **100.8** g HCOOH/water mixture (HCOOH **56.**7%, water **37.7**%) was distilled off. In the sump, **407.0** g OXE crude with precipitated ammonium sulphate was obtained. **400.5** g OXE crude with precipitated ammonium sulphate were filtered through a filter nutsch to get **6.0** g filter cake and **391.5** g OXE crude (OXE **68.7**%) as the filtrate. Yield in filtrate: **86.5**%

Washing of the reaction equipment with ethanol affords additional **5.4**% yield. Overall yield: **91.9** %

Washing of the filter cake was done twice with **11.0** g FOXE crude for each washing. After the washing **4.8** g filter cake which was free of OXE and **24.2** g filtrate (FOXE **76.9**%, OXE **2.0** %) were obtained. The filtrate can be recycled.
OXE yield saving: **0.6**%
Overall OXE yield from FOXE: **92.5**%
Overall OXE yield from EACA: **80.1**%

The FOXE remaining in the filter cake was washed from **4.4** g filter cake with **18.5** g toluene. After the washing, **3.9** g filter cake were obtained, which was free of FOXE. The **18.8** g filtrate (FOXE **8.6**%) can be recycled.

### Example 3: Rectification to purified OXE

To a thin film evaporator with a **50** cm × **30** mm Kerapak column at a temperature of **110**°C and at p = **5** mbar, **380.5** OXE crude was fed continuously. At the head of the column which is run at a reflux ratio of **0.2**, **246.1** g OXE (OXE **89.8**%) was obtained as the distillate and as a sump **67.4** g high boiling impurities (OXE **0.6**%) was obtained. Also **37.4** g hold-up was collected after the experiment.
Loss of OXE in the sump: **0.2** %
Overall yield of OXE purified from EACA: **80.0**%

## Claims

1. A process for the preparation of a compound of formula (II), which comprises reacting the compound of formula (I) with formamide in the presence of an acid catalyst, 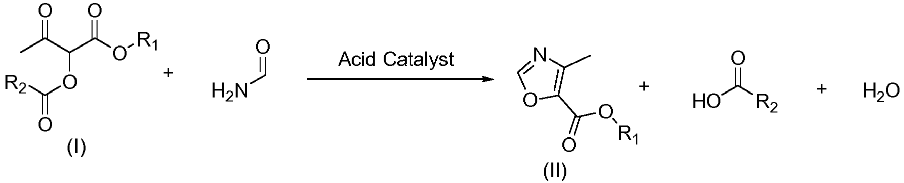 wherein: R₁ is H or C₁-C₁₀alkyl; and R₂ is H,
wherein the reaction is carried out in the absence of a solvent, and carboxylic acid and water are removed from the reaction mixture during the entire reaction.

2. The process of claim 1, wherein the compound of formula (II) is 4-methyloxazole-5-formic acid methyl ester, or 4-methyloxazole-5-formic acid ethyl ester.

3. The process of claim 1 or 2, wherein the acid catalyst is mineral acid such as H₂SO₄ or H₃PO₄, organic acid, or acidic ion exchange resin.

## Patentansprüche

1. Verfahren zur Herstellung einer Verbindung der Formel (II), bei dem man die Verbindung der Formel (I) in Gegenwart eines Säurekatalysators mit Formamid umsetzt, 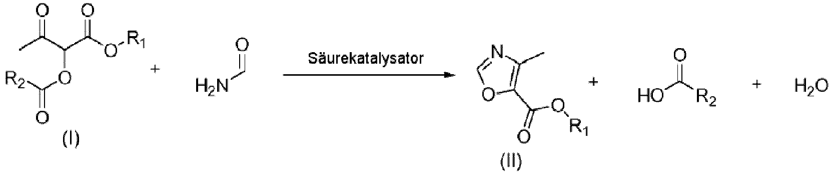 wobei: R₁ für H oder C₁-C₁₀-Alkyl steht und R₂ für H steht,
wobei die Umsetzung in Abwesenheit eines Lösungsmittels durchgeführt wird und während der gesamten Umsetzung Carbonsäure und Wasser aus der Reaktionsmischung entfernt werden.

2. Verfahren nach Anspruch 1, bei dem es sich bei der Verbindung der Formel (II) um 4-Methyloxazol-5-ameisensäuremethylester oder 4-Methyloxazol-5-ameisensäureethylester handelt.

3. Verfahren nach Anspruch 1 oder 2, bei dem es sich bei dem Säurekatalysator um Mineralsäure wie H₂SO₄ oder H₃PO₄, organische Säure oder saures Ionenaustauscherharz handelt.

## Revendications

1. Procédé de préparation d'un composé de formule (II), qui comprend la réaction du composé de formule (I) avec du formamide en présence d'un catalyseur acide, 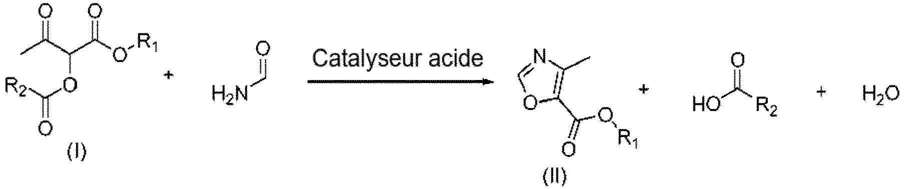 dans lequel : R₁ est H ou alkyle en C₁-C₁₀ ; et R₂ est H, dans lequel la réaction est conduite en l'absence d'un solvant, et l'acide carboxylique et l'eau sont retirés du mélange de réaction pendant la réaction entière.

2. Procédé selon la revendication 1, dans lequel le composé de formule (II) est l'ester méthylique d'acide 4-méthyloxazole-5-formique, ou l'ester éthylique d'acide 4-méthyloxazole-5-formique.

3. Procédé selon la revendication 1 ou 2, dans lequel le catalyseur acide est un acide minéral tel que H₂SO₄ ou H₃PO₄, un acide organique ou une résine d'échange d'ions acide.
